(19) Europäisches Patentamt
European Patent Office
Office européen des brevets

(11) EP 2 465 357 B1

(12) **EUROPEAN PATENT SPECIFICATION**

(45) Date of publication and mention of the grant of the patent:
**05.11.2014 Bulletin 2014/45**

(51) Int Cl.:
*A23K 1/16* (2006.01) *A23L 1/30* (2006.01)
*A23L 1/302* (2006.01) *A23L 2/52* (2006.01)
*A61K 8/34* (2006.01) *A61K 31/045* (2006.01)

(21) Application number: **11193214.1**

(22) Date of filing: **13.12.2011**

# (54) Stabilized ß cryptoxanthin-containing water and the use thereof

Stabilisiertes beta-Cryptoxanthin in Wasser und dessen Verwendung

Eau contenant de la ß cryptoxanthine stabilisée et son utilisation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2010 JP 2010279773**
**15.12.2010 JP 2010279774**
**29.11.2011 JP 2011260633**

(43) Date of publication of application:
**20.06.2012 Bulletin 2012/25**

(73) Proprietor: **Arkray, Inc.**
**Kyoto-shi, Kyoto 601-8045 (JP)**

(72) Inventors:
- **Sasaki, Takao**
**Kyoto, 602-0008 (JP)**
- **Takayama, Kiyofumi**
**Kyoto, 602-0008 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 047 760** **WO-A1-03/034841**
**WO-A1-2005/110122** **WO-A1-2007/009657**
**GB-A- 2 190 822** **US-A1- 2005 037 115**

- **DATABASE WPI Week 200957 Thomson Scientific, London, GB; AN 2009-M91561 XP002688614, & JP 2009 191002 A (FUJI SHOKKEN KK) 27 August 2009 (2009-08-27)**
- **DATABASE WPI Week 201038 Thomson Scientific, London, GB; AN 2010-F10404 XP002688615, & JP 2010 105984 A (UNITIKA LTD) 13 May 2010 (2010-05-13)**
- **DATABASE WPI Week 199530 Thomson Scientific, London, GB; AN 1995-224952 XP002688616, & CN 1 087 491 A (SHANDONG PROV FRUIT TREE INST) 8 June 1994 (1994-06-08)**
- **DHUIQUE-MAYER CLAUDIE ET AL: "Thermal degradation of antioxidant micronutrients in Citrus juice: Kinetics and newly formed compounds", JOURNAL OF AGRICULTURAL AND FOOD CHEMISTRY, vol. 55, no. 10, May 2007 (2007-05), pages 4209-4216, XP002688617, ISSN: 0021-8561, DOI: 10.1021/JF0700529**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

BACKGROUND OF THE INVENTION

The present invention relates to a stabilized β cryptoxanthin-containing water and the use thereof as food and drink additive, a method for stabilizing β cryptoxanthin and a method for producing a stabilized β cryptoxanthin-containing water.

β cryptoxanthin is a type of carotenoid and is known to exert an osteogenesis promoting effect, a lipid metabolism ameliorating effect, and a cancer suppressing effect, and the utility thereof is attracting attention (Japanese Patent No. 3892014, WO 2005/112904, and JP 2006-219388 A). In order to ingest such B cryptoxanthin having superior utility on a daily basis, it is desired that β cryptoxanthin is added as an additive substance to food and drink, for example. Oral compositions comprising cryptoxanthin and an antioxidant substance are known from JP 2010105984 and EP 2047760.

However, there is a problem in that β cryptoxanthin is unstable. Therefore, when B cryptoxanthin is added to food and drink, there is a risk that, for example, degradation thereof is progressed before being actually ingested by consumers. Further, there is no practical stabilizing method for solving the problem. Furthermore, when the β cryptoxanthin is used as an additive agent for food and drink, it is necessary that a stabilizing agent for B cryptoxanthin is a substance having no problem to be ingested on a daily basis.

US 2005/0037115 and WO3/034841 disclose stable dispersions of carotenoids and an emulsifier in an aqueous medium.

Moreover, in order to expand an application range of β cryptoxanthin as food and drink, it is desired that the β cryptoxanthin is supplied in a form of a water containing stabilized β cryptoxanthin.

BRIEF SUMMARY OF THE INVENTION

Hence, the present invention is intended to provide a β cryptoxanthin-containing water being capable of being utilized as food and drink and containing β cryptoxanthin stabilized with superior safety and a method for stabilizing β cryptoxanthin.

In order to achieve the aforementioned object, the stabilized β cryptoxanthin-containing water of the present invention is a stabilized β cryptoxanthin-containing water as defined in claim 1.

A method for stabilizing β cryptoxanthin of the present invention is the method for stabilizing β cryptoxanthin as defined in claim 4.

According to the present invention, a stabilized β cryptoxanthin-containing water having superior safety can be provided inexpensively.

BRIEF DESCRIPTION OF THE DRAWING

[FIG. 1] FIG. 1 is a graph showing an Arrhenius plot of degradation of thiamine hydrochloride in a vitamin complex liquid agent.

DETAILED DESCRIPTION OF THE INVENTION

In the present invention, stabilization of β cryptoxanthin encompasses, for example, prevention of degradation of β cryptoxanthin.

In the present invention, vitamin C to be added in order to stabilize β cryptoxanthin is referred to as "additive vitamin C".

The present invention is described below.

(1) Stabilized B cryptoxanthin-containing water

The stabilized β cryptoxanthin-containing water of the present invention is, as mentioned above, a stabilized β cryptoxanthin-containing water as defined in claim 1.

It is only necessary that the stabilized β cryptoxanthin-containing water of the present invention contains the additive vitamin C besides β cryptoxanthin in the concentrations as defined in claim 1.

As a result of the earnest studies, the inventors of the present invention found that β cryptoxanthin can be stabilized by causing additive vitamin C and β cryptoxanthin to coexist with each other in water. Vitamin C is inexpensive, and it has been proved from the history of usage, that vitamin C has superior safety to food and drink as an additive agent. Moreover, it is possible to stabilize β cryptoxanthin by vitamin C alone without using in combination with the other stabilizing agent. Therefore, according to the present invention, a stabilized β cryptoxanthin-containing water having

superior safety can be provided inexpensively. Thus, an application range of such a β cryptoxanthin-containing water containing stabilize β cryptoxanthin as food and drink such as a beverage or a food additive for food and drink is expanded, so that the 6 cryptoxanthin-containing water is very useful.

The β cryptoxanthin can be represented by the following structural formula (1). In the present invention, the β cryptoxanthin may be a derivative of a compound represented by the following structural formula (1).

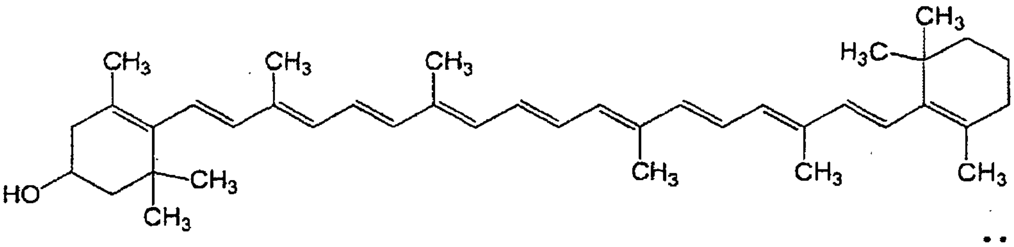

···(1)

It is known that the β cryptoxanthin represented by the structural formula (1) is generally poorly soluble in water. The form of the stabilized β cryptoxanthin-containing water is not particularly limited and is, for example, preferably an aqueous dispersion liquid in which 6 cryptoxanthin is dispersed. The dispersion encompasses suspension, for example. When a derivative of the β cryptoxanthin is soluble in water, the stabilized β cryptoxanthin-containing water may be, for example, an aqueous solution in which β cryptoxanthin is dissolved.

The β cryptoxanthin may be, for example, the one derived from a natural product or a synthetic. From the viewpoint of safety, the β cryptoxanthin is preferably the one derived from a natural product and more preferably the one derived from a plant. Examples of the plant include *Citrus, Diospyros,* and *Mangiferra.* The *Citrus* is preferably *Citrus unshiu,* the *Diospyros* is preferably *Diospyros kaki,* and the *Mangiferra* is preferably *Mangifera indica.* The *Citrus* means, for example, citrus.

The stabilized β cryptoxanthin-containing water of the present invention may be prepared by adding an single purified product of 6 cryptoxanthin to water or adding a β cryptoxanthin source containing β cryptoxanthin to water.

Examples of the vitamin C include L-ascorbic acid, L- ascorbic acid salt, and a derivative of L-ascorbic acid, and they may be used alone or in a combination of two or more of them. Examples of the L-ascorbate include alkaline metal salts and alkaline earth metal salts. Examples of the alkaline metal salts include a sodium salt and a potassium salt. Examples of the alkaline earth metal salts include a calcium salt and a magnesium salt. Examples of the derivative of L-ascorbic acid include ascorbic acid phosphate magnesium, ascorbic acid phosphate sodium, and ascorbic acid phosphate potassium.

In the stabilized β cryptoxanthin-containing water, the concentration of the vitamin C can be set without depending on a concentration of β cryptoxanthin in the stabilized B cryptoxanthin-containing water, for example. The lower limit of the concentration of the vitamin C in the stabilized β cryptoxanthin-containing water is 0.02 parts by mass or more with respect to 100 parts by mass of a combination of the water and components contained in the water. The upper limit of the concentration of the vitamin C in the stabilized β cryptoxanthin-containing water is 0.1 parts by mass or less with respect to 100 parts by mass of a combination of the water and components contained in the water. A range of the concentration of the vitamin C is from 0.02 to 0.1 parts by mass. 100 parts by mass of a combination of the water and components contained in the water can be referred to as 100 parts by mass of entire stabilized β cryptoxanthin-containing water.

The lower limit of the concentration of β cryptoxanthin in the stabilized β cryptoxanthin-containing water is 0.0015 parts by mass or more and more preferably 0.002 parts by mass or more with respect to 100 parts by mass of a combination of the water and components contained in the water. The upper limit of the concentration of β cryptoxanthin in the stabilized B cryptoxanthin-containing water is 0.006 parts by mass or less, preferably 0.0045 parts by mass or less, and more preferably 0.003 parts by mass or less with respect to 100 parts by mass of a combination of the water and components contained in the water.

Specifically, it is preferred that the concentration of β cryptoxanthin in the stabilized β cryptoxanthin-containing water is in a low concentration range, for example, from the viewpoint of cost. The low concentration range is, for example, specifically from 0.0015 to 0.006 parts by mass with respect to 100 parts by mass of the combination. When the β cryptoxanthin is in such a concentration range, the β cryptoxanthin can be particularly efficiently stabilized by the additive vitamin C. In the case where the stabilized 6 cryptoxanthin-containing water is provided as a beverage, the concentration of β cryptoxanthin is set to be in the above-described low concentration region, for example. Therefore, under such a condition, it is preferred that the stabilized 6 cryptoxanthin-containing water of the present invention is provided specifically as a beverage.

In the present invention, components contained in the water are components contained in the stabilized 6

cryptoxanthin-containing water other than the water. The components other than the water may be, for example, the β cryptoxanthin and the additive vitamin C or may further include the other component. The other component is not particularly limited, and examples thereof include an additive agent and the like added to food and drink. Examples of the additive agent include flavors, sweeteners, saccharides, acidulants, coloring agents, fruit juices, emulsifiers, dietary fibers, and vitamins. The other component may be a component contained in a β cryptoxanthin source other than the β cryptoxanthin.

The stabilized β cryptoxanthin-containing water of the present invention may be, as mentioned above, prepared by adding a single purified product of β cryptoxanthin to water or adding a β cryptoxanthin source containing β cryptoxanthin to water.

In the former case, the β cryptoxanthin is added to the water, and then the additive vitamin C is added thereto.

In the latter case, the β cryptoxanthin source is added to the water, and then the additive vitamin C is added thereto.

Examples of the β cryptoxanthin source include a fruit juice of the plant or a concentrate of the fruit juice. The β cryptoxanthin source is more preferably a fruit juice of *Citrus* or a concentrate thereof and yet more preferably a fruit juice of *Citrus unshiu* or a concentrate thereof. Therefore, the stabilized β cryptoxanthin-containing water of the present invention may be, for example, additive water obtained by adding the β cryptoxanthin source (the fruit juice or the concentrate thereof) to water and is preferably a dispersion water obtained by dispersing the β cryptoxanthin source (the fruit juice or the concentrate thereof) in water. In this case, in the stabilized β cryptoxanthin-containing water of the present invention, β cryptoxanthin is derived from, for example, a fruit juice of citrus (*Citrus*) or the concentrate thereof, preferably mandarin juice or the concentration thereof, and more preferably a fruit juice of *Citrus unshiu* or the concentrate thereof.

The β cryptoxanthin source may contain, as components other than β cryptoxanthin, for example, an excipient such as dextrin or indigestible dextrin, a flavor, a sweetener, a saccharide, an acidulant, a coloring agent, a fruit juice, an emulsifier, a dietary fiber, a vitamin, and spirits. Therefore, the stabilized β cryptoxanthin-containing water of the present invention may contain, besides the B cryptoxanthin and the additive vitamin C, the components contained in the β cryptoxanthin source other than the β cryptoxanthin as the above-mentioned "components other then the water".

In the stabilized B cryptoxanthin-containing water of the present invention, the additive vitamin C can be, for example, vitamin C derived from the β cryptoxanthin source and/or vitamin C other than the vitamin C derived from the β cryptoxanthin source. The vitamin C derived from the β cryptoxanthin source is, for example, vitamin C contained in the β cryptoxanthin source. Examples of the vitamin C other than the vitamin C derived from the β cryptoxanthin source include a single substance of vitamin C and a vitamin C composition primarily containing vitamin C. The vitamin C composition may be, for example, crude vitamin C.

It is preferred that the single substance of vitamin C is added, as the additive vitamin C, to the stabilized β cryptoxanthin-containing water of the present invention, for example. In the stabilized β cryptoxanthin-containing water of the present invention, the additive vitamin C may be, for example, vitamin C, all of which is a single substance or vitamin C, a part of which is a single substance. In the latter case, the stabilized β cryptoxanthin-containing water may contain, as the additive vitamin C, vitamin C derived from the β cryptoxanthin source, specifically vitamin C derived from a fruit juice of a plant. In the stabilized β cryptoxanthin-containing water, the additive vitamin may be, for example, vitamin C, all of which is derived from the β cryptoxanthin source (for example, vitamin C derived from a fruit juice of a plant) or vitamin C, a part of which is derived from the β cryptoxanthin source. In the present invention, a concentration of the vitamin C can easily and inexpensively be set to a desired concentration. Therefore, it is preferred that the single substance of vitamin C is added.

The β cryptoxanthin source containing β cryptoxanthin and a method for preparing the same are described using an example of *Citrus unshiu.* Note here that the same applies to the cases of *Citrus* other than *Citrus unshiu* and other plants.

In the 6 cryptoxanthin source, a fruit juice of *Citrus unshiu* can be used as a raw fruit juice, for example. Besides *Citrus unshiu,* a plant such as Japanese persimmon or *Mangifera indica,* a processed substance thereof, or the like can be used as the β cryptoxanthin source or a raw material of the β cryptoxanthin source.

The raw fruit juice may be, for example, a liquid fraction collected from whole fruit of *Citrus unshiu* or a liquid fraction collected from at least a part of the fruit of *Citrus unshiu.* Examples of the part include a pulp and a peel. β cryptoxanthin is present in various parts of the fruit, for example. Therefore, the fruit juice is preferably a liquid fraction collected from whole fruit. A method for collecting the fruit juice is not particularly limited, and for example, a conventionally known method such as expression can be employed. The fruit juice may be, for example, a filtered fruit juice.

The raw fruit juice may be collected from the *Citrus unshiu,* or a commercially available fruit juice or a concentrated fruit juice, being put into circulation may be used. The concentrated fruit juice can be, for example, a concentrated fruit juice concentrated to 1/5 to 1/7 of its original volume by a conventionally known method. The concentrated fruit juice may be, for example, the one obtained by thawing a frozen concentrated fruit juice. It is also possible that, the concentrated fruit juice is, for example, diluted with water, and the diluted fruit juice is used as the raw fruit juice.

It is preferred that the raw fruit juice is subjected to a sterilization treatment by heat in the step of producing the β cryptoxanthin source. In the sterilization treatment, various methods for heat-sterilizing a liquid substance and various devices for the same can be utilized, for example. Conditions of the sterilization treatment are not particularly limited, and a short-time treatment is preferred. The conditions can be, for example, conditions at 65°C to 95°C for 30 to 600 seconds, specifically conditions at about 95°C for about 30 seconds, or conditions under which the same sterilizing value as under the above-described conditions is exerted. By conducting a sterilization treatment in short time as described above, the bacteria count in the raw fruit juice can be controlled, and the raw fruit juice can be prevented from being decomposed in various steps described below, for example. The β cryptoxanthin is relatively stable to heating that can be generally conducted to liquid food, for example. Therefore, β cryptoxanthin in the raw fruit juice is not lost by the sterilization treatment.

After the heat treatment, the raw fruit juice is cooled to an appropriate temperature. The temperature is not particularly limited and is preferably set to a predetermined temperature that is suit on an enzyme treatment and the centrifugal treatment, being conducted after the heat treatment. The predetermined temperature is, for example, from 4°C to 37°C. The cooling is optionally conducted. A method for cooling the raw fruit juice is not particularly limited, and examples thereof include a method in which the heated raw fruit juice is filled in a container at a lower temperature compared with the raw fruit juice, which is then air-cooled or water-cooled or a method of cooling using a heat exchanger.

Thereafter, the cooled raw fruit juice is dispensed to tanks each having an appropriate volume. It is preferred that the volume of each of the tanks is set so that the enzyme treatment and the centrifugal treatment can be conducted efficiently. It is also possible that the raw fruit juice after the sterilization treatment or the raw fruit juice in the middle of the cooling is filled in the tanks, and the raw fruit juice is cooled to the predetermined temperature under the state of being filled in the tanks.

Subsequently, the cooled raw fruit juice is subjected to an enzyme treatment. The enzyme treatment is conducted optionally. It is preferred that the enzyme treatment is conducted by adding an enzyme to the raw fruit juice that has been cooled to the predetermined temperature. The enzyme is not particularly limited, and for example, pectinase, cellulase, hemicellulase, protease, lipase, a maceration enzyme, protopectinase, or the like can be used. The enzymes can be used alone or in a combination of two or more of them. In the enzyme treatment, an enzyme agent containing the enzyme can also be used. The form of the enzyme agent is not particularly limited and may be, for example, any of powder and liquid. The amount of the enzyme to be added to the raw fruit juice is not particularly limited and can be decided as appropriate according to the type of the enzyme. The conditions of the enzyme treatment are not particularly limited, and the temperature and time can be set according to the type of the enzyme. It is preferred that the enzyme is dispersed in the raw fruit juice by adding the enzyme to the raw fruit juice and then stirring them. The raw fruit juice is under the state of having low viscosity. Therefore, the enzyme can be dispersed uniformly in the entire raw fruit juice by adding the enzyme to the raw fruit juice and then stirring them. The enzyme treatment is conducted for a predetermined time under the state where the raw fruit juice is at a temperature that is appropriate for the enzyme according to the type of the enzyme. The amount of the β cryptoxanthin in the raw fruit juice does not increase or decrease by the enzyme treatment itself.

The raw fruit juice obtained after being subjected to the enzyme treatment as it is may be subjected to a centrifugal treatment or may be subjected to a step of finishing the enzyme reaction by heating again before the centrifugal treatment.

The raw fruit juice obtained after the enzyme treatment is subjected to centrifugal separation. The centrifugal separation may be conducted to the raw fruit juice that is obtained after the cooling and has not been subjected to an enzyme treatment, for example. By concentrating the raw fruit juice through the centrifugal separation, the raw fruit juice can be separated into a liquid pulp fraction being high in β cryptoxanthin and a supernatant fraction being low in β cryptoxanthin. A centrifugal separator used for the centrifugal separation is not particularly limited, and any of commercially available various centrifugal separators can be used. The flow rate in the centrifugal separation is, for example, about 4 to 5 t/hour. The number of times that the centrifugal separation is conducted may be, for example, one to the raw fruit juice or plural times after conducting membrane treatment such as ultrafiltration, after changing centrifugal intensity, and the like. A liquid fraction being low in β cryptoxanthin is separated from the raw fruit juice by the membrane treatment such as ultrafiltration, and a liquid pulp residue in which β cryptoxanthin is concentrated can be obtained.

The liquid pulp in which β cryptoxanthin is concentrated, being obtained by the centrifugal separation as it is can be used as a concentrated liquid that is a β cryptoxanthin source, for example. The liquid pulp may further be concentrated in order to form into a paste, or a powder. It is also possible that the concentrated liquid, the paste, or the powder is caused to be in contact with an organic solvent so as to be an extract in which β cryptoxanthin is concentrated.

The forming the liquid pulp into a concentrated liquid or a paste is conducted by concentrating the liquid pulp by a conventionally known concentration method such as vacuum drying, for example. Specifically, for example, the pulp fraction is concentrated by removing the moisture thereof through vacuum drying. Thus, a concentrated liquid containing β cryptoxanthin or a paste containing the same can be obtained.

The forming the liquid pulp into a powder is conducted by treating the liquid pulp by a conventionally known

method such as freeze-drying or spray drying. When the pulp fraction is formed into a powder, it is preferred that, a commercially available excipient is added to the powder, for example. As the excipient, any of starch, various dextrins, cyclic dextrin, trehalose, lactose, oligosaccharide, sucrose ester, and fatty acid ester can be used. The concentration of β cryptoxanthin in the powder is reduced as the amount of the excipient to be added is increased. Therefore, it is preferred that the amount of the excipient to be added is the minimum amount, for example.

The extract can be obtained by, for example, causing the concentrated liquid (the liquid pulp), the paste, the powder, or the like to be in contact with an organic solvent, then collecting the organic solvent, and thereafter removing a part or a whole of the organic solvent. As the organic solvent, ethanol, hexane, or the like can be used, for example. The organic solvents may be used alone or in a combination of two or more of them.

The concentrate such as the concentrated liquid, the paste, the powder, the extract, or the like obtained in the above-described manner can be used as a β cryptoxanthin source. In production of the stabilized β cryptoxanthin-containing water of the present invention, it is preferred that the concentrate is dispersed in water, for example. When the concentrate is dispersed in water, a dispersant may further be added thereto, for example. When the extract extracted with the organic solvent is dispersed in water, it is specifically preferred that a dispersant is added thereto, for example. As the dispersant, ethanol, an emulsifier, or the like can be used, for example. The emulsifier is not particularly limited, and a conventionally known emulsifier can be used. The emulsifier is specifically preferably an emulsifier for food. As the emulsifier, any of, for example, glycerin fatty acid ester (monoglyceride), organic acid monoglyceride, polyglycerin fatty acid ester (polyglycerin ester), polyglycerol condensed ricinoleic acid ester, saponin, sucrose fatty acid ester (sucrose ester), lecithin, enzymatically decomposed lecithin, sorbitan fatty acid ester, and propylene glycol fatty acid ester; vegetable gums such as gum arabic and xanthan gum; starch such as dextrin and processed starch; and proteins such as casein and gelatin can be used. The emulsifier is preferably an emulsifier having a HLB value of 10 or more among them, and the emulsifier having a HLB value of 10 or more can be, for example, polyglycerin fatty acid ester, sucrose fatty acid ester, or the like. Emulsifying the extract with such an emulsifier is preferable in order to improve bioavailability, for example.

The stabilized β cryptoxanthin-containing water obtained by stabilizing β cryptoxanthin in any of the various concentrates obtained as described above can be prepared by causing any of the various concentrates as a β cryptoxanthin source to coexist with the additive vitamin C in water.

When the stabilized β cryptoxanthin-containing water of the present invention is produced under the condition under which the stabilized β cryptoxanthin-containing water contains 0.002 parts by mass of β cryptoxanthin and 0.01 parts by mass of the vitamin C, which is then stored at 40°C, degradation of β cryptoxanthin can be suppressed for about two months. Specifically, a residual ratio (%) of β cryptoxanthin can be maintained to, for example, 80% or more, preferably 90% or more, and more preferably 95% or more, assuming that the amount of β cryptoxanthin in the stabilized β cryptoxanthin-containing water in production is 100%.

(2) Food and drink additive

The use of the stabilized β cryptoxanthin-containing water as food and drink additive is described in claim 2. Examples of the food and drink include beverages and food products. Examples of the beverages include beverages containing no fruit juice, beverages containing fruit juice, lactic acid bacteria beverages, milk beverages, and powder beverages. Examples of the food products include: frozen desserts such as ice creams, sherbet, and ice desserts; dessert food products such as pudding, jelly, Bavarian creams, and yoghurts.

The use of the stabilized β cryptoxanthin-containing water is not limited to food and drink, and it can be utilized in, for example, pharmaceuticals, quasi drugs, cosmetics, feed, food additives, and the like besides food and drink.

(3) β cryptoxanthin stabilizing method

In the β cryptoxanthin stabilizing method of the present invention, β cryptoxanthin is stabilized by causing the β cryptoxanthin to coexist with vitamin C in water.

The vitamin C is artificially caused to coexsist with β cryptoxanthin and corresponds to the additive vitamin C in the stabilized β cryptoxanthin-containing water of the present invention. The β cryptoxanthin stabilizing method of the present invention can be cited from the explanation of the stabilized β cryptoxanthin-containing water of the present invention unless otherwise shown.

In the present invention, a method for causing β cryptoxanthin and the additive vitamin C to coexist with each other in water is not particularly limited. The order of adding water, β cryptoxanthin, and the additive vitamin C is not particularly limited. For example, it may be possible that the β cryptoxanthin and the additive vitamin C are added to water at the same time; the β cryptoxanthin is added to water, and then the additive vitamin C is added thereto; or the additive vitamin C is added to water, and then the β cryptoxanthin is added thereto.

In the present invention, it is also possible that β cryptoxanthin and the additive vitamin C are caused to coexist

with each other in water using the β cryptoxanthin source containing B cryptoxanthin. In this case, the β cryptoxanthin source and the additive vitamin C may be added to the water. The order of adding the β cryptoxanthin source and the additive vitamin C is not particularly limited. For example, it may be possible that the β cryptoxanthin source and the additive vitamin C are added to water at the same time; the β cryptoxanthin source is added to water, and then the additive vitamin C is added thereto; or the additive vitamin C is added to water, and then the β cryptoxanthin source is added thereto. It is preferred that the β cryptoxanthin source is dispersed in water, for example.

The present invention includes, for example, adjusting the concentration of vitamin C in the water. The concentration of vitamin C in the water is as mentioned above. The adjusting the concentration of vitamin C may be conducted by adding the β cryptoxanthin source or vitamin C other than the β cryptoxanthin source.

(4) Stabilized β cryptoxanthin-containing water producing method

The stabilized β cryptoxanthin-containing water producing method of the present invention includes: stabilizing β cryptoxanthin in water, and the stabilizing is conducted by the stabilizing method of the present invention. In the producing method of the present invention, the stabilizing method of the present invention is conducted, and the other steps and conditions are not at all limited. The producing method of the present invention can be cited from, for example, the explanations of the stabilized β cryptoxanthin-containing water of the present invention and the stabilizing method of the present invention.

In the other aspect, the stabilized β cryptoxanthin-containing water of the present invention is obtained by the stabilized β cryptoxanthin-containing water producing method of the present invention.

Examples

The examples of the present invention are described below.

[Example 1A]

(1) Preparation of β cryptoxanthin-containing paste

A concentrated fruit juice of *Citrus unshiu* was diluted with water. Thus, a fruit juice from concentrate was prepared. The fruit juice from concentrate was subjected to a sterilization treatment at 95°C for 30 seconds and was then subjected to a centrifugal treatment. Thus, the fruit juice from concentrate was separated into a liquid pulp fraction being high in β cryptoxanthin and a supernatant fraction being low in β cryptoxanthin. Then moisture of the obtained liquid pulp fraction was removed by concentrating it through vacuum drying. Thus, a β cryptoxanthin-containing paste was obtained. The paste had a moisture percentage of 60% and contained 1.3 w/w% of dietary fiber, 0.07 w/w% of β cryptoxanthin, and 0.006 w/w% of vitamin C.

(2) Preparation of β cryptoxanthin-containing water

The prepared paste derived from *Citrus unshiu* and water were mixed. Thus, a mixed solution was prepared. The mixing was conducted so that a mass ratio between β cryptoxanthin contained in the paste and the mixed water became 1 mg : 50 g (0.002 parts by mass of β cryptoxanthin with respect to 100 parts by mass of finally obtained β cryptoxanthin-containing water). Vitamin C (L-ascorbic acid) was added to the mixed solution, which was then mixed by stirring. Thus, β cryptoxanthin-containing waters were prepared. The total amounts of L-ascorbic acid in the respective β cryptoxanthin-containing waters were set to 0.005, 0.01, 0.02, 0.05, 0.1, and 0.2 parts by mass with respect to 100 parts by mass of entire β cryptoxanthin-containing water by adding vitamin C. As a blank, a β cryptoxanthin-containing water was prepared in the same manner as described above except that vitamin C (L-ascorbic acid) was not added to the mixed solution.

(3) β cryptoxanthin stabilizing test

50 g of six types of the β cryptoxanthin-containing waters and 50 g of the β-cryptoxanthin-containing water as a blank were filled in the respective brown bottles. Thereafter, the brown bottles were subjected to a heat sterilization treatment under the condition at 90°C for 30 minutes, which were then stored at 40°C. Sampling was conducted on a storage start date, after one month from the storage start date, and after two months from the same. The respective amounts of β cryptoxanthin in samples were measured by HPLC as shown below. Then, assuming that the amount of β cryptoxanthin on the storage start date was 100%, residual ratios of β cryptoxanthin were determined.

The samples were treated at 70°C for 30 minutes under alkaline conditions before being subjected to HPLC.

By this treatment, β cryptoxanthin in the samples was saponified and was converted to a free form. After the treatment, the samples were subjected to a liquid-liquid extraction and were then concentrated. Thus, samples for HPLC were obtained.

In the measurements of the amounts of β cryptoxanthin by HPLC, a HPLC system to which a column (J'sphere ODS-M80 (product name) with 250 x 4.6 mm I.D, produced by YMC Co., Ltd.) had been connected was used, and absorbances at 455 nm were measured by a UV detector (L-4200H (product name), produced by Hitachi Ltd.). Conditions of the column were as follows.

Mobile phase: acetonitrile/tetrahydrofuran (volume ratio: 95/5) containing 0.1% acetic acid and 50 ppm of α-tocopherol
Flow rate: 1.0 mL/minute
Column temperature: 55°C

A relationship between the concentration of the additive L-ascorbic acid and the residual ratio of the β cryptoxanthin is shown in Table 1.

As shown in Table 1, it was confirmed that β cryptoxanthin was stabilized by causing β cryptoxanthin to coexist with additive L-ascorbic acid in water. Specifically, when the concentration of the additive L-ascorbic acid was 0.01 parts by mass or more with respect to 100 parts by mass of a combination of the water and components contained in the water, the β cryptoxanthin was more stable.

[Table 1]

| Concentration of additive L-ascorbic acid (parts by mass) | Residual ratio of β cryptoxanthin | |
|---|---|---|
| | Storage for one month (%) | Storage for two months (%) |
| 0 (*) | 76.9 | 29.9 |
| 0.005 (*) | 83.8 | 48.8 |
| 0.01 (*) | 96.7 | 78.9 |
| 0.02 | 100 | 100 |
| 0.05 | 98.0 | 99.0 |
| 0.1 | 94.4 | 94.7 |
| 0.2 (*) | 68.3 | 31.3 |

(*) Reference Example

[Example 2A]

(1) Preparation of B cryptoxanthin-containing powder

A concentrated fruit juice of *Citrus unshiu* was diluted with water. Thus, a fruit juice from concentrate was prepared. The fruit juice from concentrate was subjected to a sterilization treatment at 95°C for 30 seconds and was then subjected to a centrifugal treatment. Thus, the fruit juice from concentrate was separated into a liquid pulp fraction being high in β cryptoxanthin and a supernatant fraction being low in β cryptoxanthin. Then moisture of the obtained liquid pulp fraction was removed by concentrating it through vacuum drying. Thus, a paste was obtained. A diluent was added to the paste, which was then subjected to spray drying. Thus, a β cryptoxanthin-containing powder was obtained. The powder had a moisture percentage of 2.2% and contained 9.1 w/w% of protein, 9.4 w/w% of lipid, 75.6 w/w% of carbohydrate, and 0.136 w/w% of β cryptoxanthin.

(2) Preparation of β cryptoxanthin-containing water

The prepared powder derived from *Citrus unshiu* and water were mixed. Thus, a mixed solution was prepared. The mixing was conducted so that a mass ratio between β cryptoxanthin contained in the powder and the mixed water became 1 mg : 50 g (0.0022 parts by mass of β cryptoxanthin with respect to 100 parts by mass of finally obtained β cryptoxanthin-containing water). Vitamin C (L-ascorbic acid) was added to the mixed solution, which was then mixed by stirring. Thus, β cryptoxanthin-containing waters were prepared. The total amounts of L-ascorbic acid in the respective β cryptoxanthin-containing waters were set to 0.005, 0.01, 0.02, 0.1, and 0.2 parts by mass with respect to 100 parts by mass of entire β cryptoxanthin-containing water by adding vitamin C.

(3) β cryptoxanthin stabilizing test

50 g of five types of the β cryptoxanthin-containing waters were filled in the respective brown bottles. Thereafter, the brown bottles were subjected to a heat sterilization treatment under the condition at 90°C for 30 minutes, which were then stored at 40°C. Sampling was conducted on a storage start date and after two months from the same. The respective amounts of β cryptoxanthin in samples were measured in the same manner as in Example 1A. Then, assuming that the amount of β cryptoxanthin on the storage start date was 100%, residual ratios of β cryptoxanthin were determined.

A relationship between the concentration of the additive L-ascorbic acid and the residual ratio of the β cryptoxanthin is shown in Table 2.

As shown in Table 2, it was confirmed that β cryptoxanthin was stabilized by causing β cryptoxanthin to coexist with additive L-ascorbic acid in water. Specifically, when the concentration of the additive L-ascorbic acid was 0.02 parts by mass or more with respect to 100 parts by mass of a combination of the water and components contained in the water, the β cryptoxanthin was more stable.

[Table 2]

| Concentration of additive L-ascorbic acid (parts by mass) | Residual ratio of β cryptoxanthin Storage for two months (%) |
|---|---|
| 0.005 (*) | 62.5 |
| 0.01 (*) | 75.5 |
| 0.02 | 92.0 |
| 0.1 | 100 |
| 0.2 | 31.9 |
| (*) Reference Example | |

[Example 3A]

(1) Preparation of concentrated liquid containing β cryptoxanthin

A concentrated fruit juice of *Citrus unshiu* was diluted with water. Thus, a fruit juice from concentrate was prepared. The fruit juice from concentrate was subjected to a sterilization treatment at 95°C for 30 seconds and was then subjected to a centrifugal treatment. Thus, the fruit juice from concentrate was separated into a liquid pulp fraction being high in β cryptoxanthin and a supernatant fraction being low in β cryptoxanthin. Then the obtained liquid pulp fraction was concentrated to 1/2 of its original volume through vacuum drying. Thus, a concentrated liquid containing β cryptoxanthin was obtained. The concentrated liquid had a moisture percentage of 80% and contained 2.9 w/w% of protein, 0.3 w/w% of lipid, 15 w/w% of carbohydrate, 2.5 w/w% of dietary fiber, and 0.03 w/w% of β cryptoxanthin.

(2) Preparation of β cryptoxanthin-containing water

The prepared concentrated liquid derived from *Citrus unshiu* and water were mixed. Thus, a mixed solution was prepared. The mixing was conducted so that a mass ratio between β cryptoxanthin contained in the concentrated liquid and the mixed water became 1 mg : 50 g (0.0015 parts by mass of β cryptoxanthin with respect to 100 parts by mass of finally obtained β cryptoxanthin-containing water). Vitamin C (L-ascorbic acid) was added to the mixed solution, which was then mixed by stirring. Thus, β cryptoxanthin-containing waters were prepared. The total amounts of L-ascorbic acid in the respective β cryptoxanthin-containing waters were set to 0.005, 0.01, 0.02, 0.1, and 0.2 parts by mass with respect to 100 parts by mass of entire β cryptoxanthin-containing water by adding vitamin C.

(3) β cryptoxanthin stabilizing test

50 g of five types of the β cryptoxanthin-containing waters were filled in the respective brown bottles. Thereafter, the brown bottles were subjected to a heat sterilization treatment under the condition at 90°C for 30 minutes, which were then stored at 40°C. Sampling was conducted on a storage start date and after two months from the same. The respective amounts of β cryptoxanthin in samples were measured in the same manner as in Example 1A. Then, assuming that the amount of β cryptoxanthin on the storage start date was 100%, residual ratios of β cryptoxanthin were determined.

A relationship between the concentration of the additive L-ascorbic acid and the residual ratio of the β cryptoxanthin is shown in Table 3.

As shown in Table 3, it was confirmed that β cryptoxanthin was stabilized by causing β cryptoxanthin to coexist

with additive L-ascorbic acid in water. Specifically, when the concentration of the additive L-ascorbic acid was 0.02 parts by mass or more with respect to 100 parts by mass of a combination of the water and components contained in the water, the β cryptoxanthin was more stable.

[Table 3]

| Concentration of additive L-ascorbic acid (parts by mass) | Residual ratio of β cryptoxanthin Storage for two months (%) |
|---|---|
| 0.005 (*) | 64.3 |
| 0.01 (*) | 64.9 |
| 0.02 | 99.1 |
| 0.1 | 100 |
| 0.2 (*) | 37.9 |

(*) Reference Example

[Example 4A]

With respect to each of the β cryptoxanthin-containing waters prepared in Examples 1A, 2A, and 3A, storage stability at 20°C and 25°C was determined.

The storage stability was calculated by the following method with reference to the publication below. Conditions were described in the publication, hereby incorporated herein by reference.

Sumie Yoshioka (1995), "Iyakuhin no Anzensei-yoriyoi Kaihatsu to Hyoka notameno Kiso kara Jissai made-(Drug safety- principles and practices for improving development and evaluation-), Nankodo Co., Ltd.

β cryptoxanthin is generally converted into vitamin A and is thus categorized as provitamin A. Hence, stability of β cryptoxanthin was predicted, i.e., the stable periods of 6 cryptoxanthin at 20°C and 25°C were calculated based on the activation energy of vitamin assuming that the activation energy of β cryptoxanthin is approximates to the activation energy of vitamin. Specifically, a graph of FIG. 185 on page 95 of the publication was cited. The graph is shown in FIG. 1. FIG. 1 is an Arrhenius plot of degradation of thiamine hydrochloride in a vitamin complex liquid agent, the Y axis indicates log $K_{obs}$ ($day^{-1}$), and the X axis indicates 1/T ($K^{-1}$), The stable periods were calculated based on the activation energy for degradation of thiamine hydrochloride in the vitamin complex liquid agent, shown in the graph.

In FIG. 1, the Y axis is indicated by not In, but log. Therefore log $K_{obs}$ was converted into In k. $K_{obs}$, $log_{10}k$, T (°C), and 1/T ($K^{-1}$) obtained based on the plotted data of FIG. 1 are shown in Table 4 below. From these data in Table 4, log $K_{obs}$ on the Y axis was converted into In k. The results obtained by the conversion of log $K_{obs}$ into In k also are shown in Table 4 below.

[Table 4]

| $K_{obs}$ | $log_{10}k$ | T (°C)*1 | 1/T ($K^{-1}$)*2 | In k |
|---|---|---|---|---|
| 0.050 | -1.300 | 67.7 | 0.00294 | -2.99336 |
| 0.017 | -1.780 | 57.6 | 0.00303 | -4.0986 |
| 0.005 | -2.300 | 50.0 | 0.00310 | -5.29595 |
| 0.001 | -2.900 | 38.7 | 0.00321 | -6.6775 |
| 0.000 | -3.400 | 29.1 | 0.00331 | -7.82879 |
| 0.000 | -3.750 | 22.5 | 0.00338 | -8.63469 |
| 0.000 | -4.100 | 20.0 | 0.00341 | -9.4406 |

*1: Celsius (C)
*2: values obtained by converting Celsius (C) into Kelvin (K)

$$K = C + 273.15$$

Then, $-E_a/R$ and In A were calculated from the In k and 1/T ($K^{-1}$) on the X axis of FIG. 1 based on the Arrhenius

equation represented by the formula (1) below. The results showed that $-E_a/R$ satisfied a = -13005, and In A satisfied b = 35.139.

$$y = ax + b \quad \cdots(1)$$

$$y = \ln k$$

$$x = 1/T$$

$$a = -E_a/R$$

$$b = \ln A$$

Subsequently, possible storage periods at 20°C and 25°C were calculated using the following formula (2) described on page 142 of the publication from the the obtained $-E_a/R$ and storage results (obtained after storage at 40°C for 2 months) of Example 1A, 2A, and 3A.

$$\ln t_{90(T1)}/t_{90(T2)} = (E_a/R)(1/T_1 - 1/T_2) \quad \cdots (2)$$

It was determined that the B cryptoxanthin-containing waters of Example 1A, 2A, and 3A can store β cryptoxanthin in the stable state for about 34 months under the condition of 20°C and for about 16.2 months under the condition of 25°C. This result suggests that β cryptoxanthin can be stored in a stable state for two years or more at normal temperature (20°C to 25°C), for example.

As described above, in the stabilized B cryptoxanthin-containing water of the present invention, ß cryptoxanthin was stabilized by adding vitamin C to a B cryptoxanthin-containing water. Vitamin C is inexpensive and has superior safety to food and drink as an additive agent. Moreover, 6 cryptoxanthin can be stabilized by vitamin C without using in combination with the other stabilizing agent. Therefore, according to the present invention, a stabilized B cryptoxanthin-containing water having superior safety can be provided with low cost. Thus, a range of application of the stabilized β cryptoxanthin-containing water is expanded to food and drink such as a beverage or a food additive for food and drink, so that the stabilized B cryptoxanthin-containing water is very useful.

## Claims

**1.** A stabilized β cryptoxanthin-containing water comprising:

water;
β cryptoxanthin; and
vitamin C, wherein
the β cryptoxanthin is stabilized by adding the vitamin C to the water containing the β cryptoxanthin,

wherein the concentration of the vitamin C in the water is from 0.02 parts by mass or more to 0.1 parts by mass or less with respect to 100 parts by mass of a combination of the water and components contained in the water, and the concentration of the β cryptoxanthin in water is from 0.0015 parts by mass or more to 0.006 parts by mass or less with respect to 100 parts by mass of a combination of the water and components contained in the water.

**2.** Use of the stabilized β cryptoxanthin-containing water according to claim 1 as food and drink additive.

**3.** The use of the stabilized β cryptoxanthin-containing water according to claim 2, wherein the β cryptoxanthin is derived from *Citrus unshiu.*

**4.** A method for stabilizing β cryptoxanthin, wherein
β cryptoxanthin is stabilized by causing the β cryptoxanthin and vitamin C to be in water and, wherein
the concentration of the vitamin C in the water is 0.02 parts by mass or more to 0.1 parts by mass or less with respect

to 100 parts by mass of a combination of the water and components contained in the water, and
the concentration of the β cryptoxanthin in water is from 0.0015 parts by mass or more to 0.006 parts by mass or less with respect to 100 parts by mass of a combination of the water and components contained in the water.

**5.** A method for producing a stabilized β cryptoxanthin-containing water, comprising:

stabilizing β cryptoxanthin in water, wherein
the stabilizing is performed by the method according to claim 4.

**Patentansprüche**

**1.** Stabilisiertes β-Cryptoxanthin enthaltendes Wasser, umfassend:

Wasser;
β-Cryptoxanthin; und
Vitamin C, worin
das β-Cryptoxanthin durch Zugeben des Vitamins C zu dem Wasser, das das β-Cryptoxanthin enthält, stabilisiert ist,
worin die Konzentration des Vitamins C in dem Wasser von 0,02 Masseteile oder mehr bis 0,1 Masseteile oder weniger in Bezug auf 100 Masseteile einer Kombination des Wassers und der Komponenten, die in dem Wasser enthalten sind, beträgt, und worin die Konzentration des β-Cryptoxanthins in dem Wasser von 0,0015 Masseteile oder mehr bis 0,006 Masseteile oder weniger in Bezug auf 100 Masseteile einer Kombination des Wassers und der Komponenten, die in dem Wasser enthalten sind, beträgt.

**2.** Verwendung des stabilisierten, β-Cryptoxanthin enthaltenden Wassers gemäß Anspruch 1 als Speisen- und Getränkeadditiv.

**3.** Verwendung des stabilisierten, β-Cryptoxanthin enthaltenden Wassers gemäß Anspruch 2, worin das β-Cryptoxanthin aus *Citrus unshiu* erhalten ist.

**4.** Verfahren zum Stabilisieren von β-Cryptoxanthin, worin β-Cryptoxanthin dadurch stabilisiert wird, dass das β-Cryptoxanthin und Vitamin C in Wasser vorliegen, und
worin die Konzentration des Vitamins C in dem Wasser 0,02 Masseteile oder mehr bis 0,1 Masseteile oder weniger in Bezug auf 100 Masseteile einer Kombination des Wassers und der Komponenten, die in dem Wasser enthalten sind, beträgt und
worin die Konzentration des β-Cryptoxanthins in dem Wasser von 0,0015 Masseteile oder mehr bis 0,006 Masseteile oder weniger in Bezug auf 100 Masseteile einer Kombination des Wassers und der Komponenten, die im Wasser enthalten sind, beträgt.

**5.** Verfahren zur Herstellung von stabilisierten, β-Cryptoxanthin enthaltendem Wasser, umfassend:

Stabilisieren des β-Cryptoxanthins in Wasser, worin das Stabilisieren durch das Verfahren gemäß Anspruch 4 durchgeführt wird.

**Revendications**

**1.** Eau contenant de la β-cryptoxanthine stabilisée comprenant :

de l’eau ;
de la β cryptoxanthine ; et
de la vitamine C, dans laquelle
la β cryptoxanthine est stabilisée par l’ajout de la vitamine C à l’eau contenant de la β cryptoxanthine,
dans laquelle la concentration de la vitamine C dans l’eau se trouve dans la plage allant de 0,02 partie en masse ou plus à 0,1 partie en masse ou moins par rapport à 100 parties en masse d’une combinaison de l’eau et de composants contenus dans l’eau, et
la concentration de la β cryptoxanthine dans l’eau se trouve dans la plage allant de 0,0015 partie en masse ou

plus à 0,006 partie en masse ou moins par rapport à 100 parties en masse d'une combinaison de l'eau et de composants contenus dans l'eau.

**2.** Utilisation de l'eau contenant de la β-cryptoxanthine stabilisée selon la revendication 1 comme additif alimentaire et additif de boisson.

**3.** Utilisation de l'eau contenant de la β-cryptoxanthine stabilisée selon la revendication 2, dans laquelle la β cryptoxanthine est dérivée de *Citrus unshiu.*

**4.** Procédé de stabilisation de la β cryptoxanthine, dans lequel
la β cryptoxanthine est stabilisée en amenant la β cryptoxanthine et la vitamine C à être dans l'eau et, dans laquelle la concentration de la vitamine C dans l'eau se trouve dans la plage allant de 0,02 partie en masse ou plus à 0,1 partie en masse ou moins par rapport à 100 parties en masse d'une combinaison de l'eau et de composants contenus dans l'eau, et
la concentration de la β cryptoxanthine dans l'eau se trouve dans la plage allant de 0,0015 partie en masse ou plus à 0,006 partie en masse ou moins par rapport à 100 parties en masse d'une combinaison de l'eau et de composants contenus dans l'eau.

**5.** Procédé de production d'une eau contenant de la β cryptoxanthine stabilisée, comprenant le fait :

de stabiliser la β cryptoxanthine dans l'eau, dans lequel
la stabilisation est réalisée par le procédé selon la revendication 4.

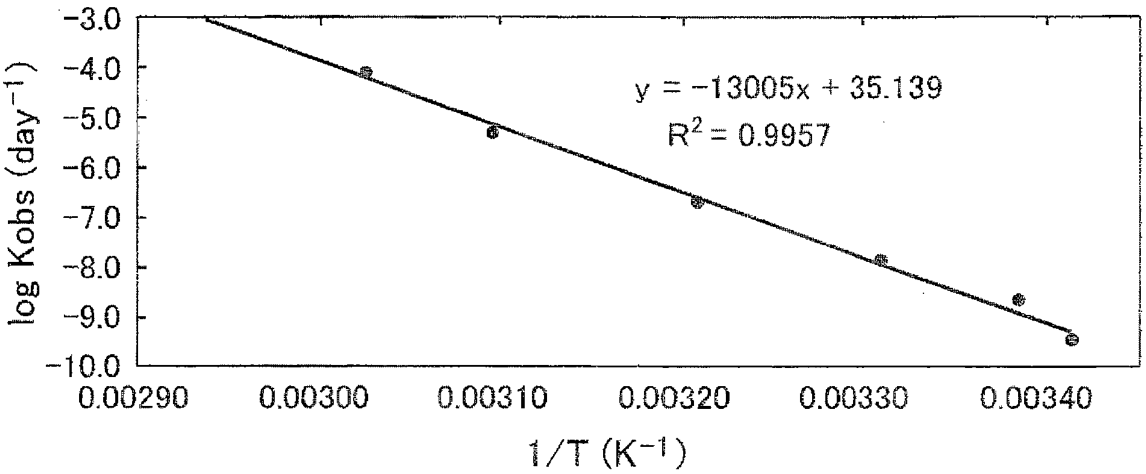

y = −13005x + 35.139
$R^2$ = 0.9957
log Kobs (day$^{-1}$)
−3.0
−4.0
−5.0
−6.0
−7.0
−8.0
−9.0
−10.0
0.00290
0.00300
0.00310
0.00320
0.00330
0.00340
1/T (K$^{-1}$)


FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3892014 B
- WO 2005112904 A
- JP 2006219388 A
- JP 2010105984 B
- EP 2047760 A
- US 20050037115 A
- WO 3034841 A

### Non-patent literature cited in the description

- **SUMIE YOSHIOKA.** Iyakuhin no Anzensei-yoriyoi Kaihatsu to Hyoka notameno Kiso kara Jissai made. Nankodo Co., Ltd, 1995